# EUROPEAN PATENT SPECIFICATION

(11) **EP 1 317 543 B1**
(45) Date of publication and mention of the grant of the patent: **02.03.2005**
(21) Application number: 01972467.3
(22) Date of filing: 11.09.2001
(51) Int. Cl.: C12N 15/28, C12N 15/70, C07K 14/415, A61K 39/36, G01N 33/68

(54) **PARIETARIA JUDAICA NS-LTP ANTIGEN VARIANTS, USES THEREOF AND COMPOSITIONS COMPRISING THEM**
PARIETARIA JUDAICA NS-LTP-ANTIGENVARIANTEN, ENTSPRECHENDE PHARMAZEUTISCHE ZUSAMMENSETZUNGEN UND IHRE VERWENDUNG
VARIANTS DE L'ANTIGENE NS-LTPS PARIETARIA JUDAICA, LEURS UTILISATIONS ET COMPOSITIONS LES COMPRENANT

(30) Priority: 11.09.2000 IT RM000494
(43) Date of publication of application: 11.06.2003
(73) Proprietor: CONSIGLIO NAZIONALE DELLE RICERCHE, 00185 Roma (IT)
(72) Inventor: GERACI, Domenico, I-90144 Palermo (IT); COLOMBO, Paolo, I-90146 Palermo (IT); DURO, Giovanni, I-90145 Palermo (IT); IZZO, Vincenzo, I-90121 Palermo (IT); COSTA, Maria, Assunta, I-90144 Palermo (IT)
(74) Representative: Germinario, Claudio
(86) International application number: PCT/IT2001/000471
(87) International publication number: WO 2002/020790

(56) References cited:
- WO-A-00/44781
- COLOMBO P. ET AL: "Identification of an immunodominant IgE epitope of the Parietaria judaica major allergen" JOURNAL OF IMMUNOLOGY, vol. 160, no. 6, 15 March 1998 (1998-03-15), pages 2780-5, XP002186915
- FERREIRA F. ET AL: "Modulation of IgE reactivity of allergens by site-directed mutagenesis: potential use of hypoallergenic variants for immunotherapy" FASEB J, vol. 12, February 1998 (1998-02), pages 231-242, XP002186916
- OLSSON S. ET AL : "Contribution of disulphide bonds to antigenicity of Lep d 2, the major allergen of the dust mite Lepidoglyphus destructor" MOLECULAR IMMUNOLOGY, vol. 35, 1998, pages 1017-1023, XP001026605
- BONURA A. ET AL: "Hypoallergenic variants of the Parietaria judaica makor allergen Par j 1: a member of the non-specific lipid transfer protein plant family" INT ARCH ALLERGY IMMUNOL, vol. 126, no. 1, September 2001 (2001-09), pages 32-40, XP001037388

## Description

### Field of the invention

The present invention relates to the fields of the prevention and the treatment of allergic symptoms associated with allergens belonging to the non-specific Lipid Transfer Protein (ns-LTPs) family.

### State of the art

Ns-LTPs proteins are small proteic molecules of approximately 10 KDa that demonstrate high stability, and are naturally present in all vegetal organisms studied to date. In several species they have also been identified as allergens, as in the case of the Rosaceae Prunoideae (peach, apricot, plum) and Pomoideae (apple), and Graminaceae, as in the Urticaceae like Parietaria Judaica (18-23).

These proteins are characterized by their ability to transport lipids through membranes in vitro, an ability justifying their denomination and corresponding to at least some of the activities exerted in vivo (17).

However, in spite of the different functions and of the heterogeneity of their sequence, ns-LTPs have a highly conserved secondary structure, comprising four alpha-helices (separated by loops) and one folded beta layer arranged in the 5'-3' direction according to a α-α-α-α-β pattern.

This structure is provided by the presence of four disulfide bridges formed by eight cysteine residues present in the 4 alpha-helices in the fourth loop, in the folded beta-layer and in the amino-terminal region (cfr. ref. 29).

In particular:
- a first disulfide bridge connects the amino-terminal region and the third alpha-helix,
- a second disulfide bridge connects the first alpha-helix and the third alpha-helix,
- a third disulfide bridge connects the second alpha-helix and the fourth loop, and
- a fourth disulfide bridge connects the third alpha-helix and the folded beta-layer.

These cysteine residues are highly conserved in all ns-LTPs, and with reference thereto a consensus sequence can be derived (17).

Despite their high conservation, given the sequence heterogeneity of ns-LTPs, no notation system for the residues forming disulfide bridges valid for all ns-LTPs exists, though those skilled in the art may easily single out such cysteines using the knowledge of the state of the art.

With particular reference to the Parj1 protein, and specifically to the mature ParJ1.0102 form, the cysteines apt to form disulfide bridges are the residues 4, 14, 29, 30, 50, 52, 75 and 91, and the related bridges are arranged in the order Cys4- Cys52 (first bridge), Cys14-Cys29 (second bridge), Cys30- Cys75 (third bridge), Cys50- Cys91 (fourth bridge) (12).

Par J1, besides being an ns-LTP, represents, together with Par J2, one of the major allergens of the Parietaria Judaica (PJ), a plant whose pollen constitutes one of the most widespread environmental antigens, especially in the Mediterranean area (1).

In fact, Parietaria Judaica pollen contains at least nine allergens with molecular masses ranging from 10 to 80 KDa and different capabilities of binding IgE [1-9].

Thereamong, Parj1, in the two isoform par11.1.2 and Parj1.0201 isolated from independent genes (10) and Parj2 acquire a remarkable relevance as major allergens. In particular these two ns-LTPs are capable of inhibiting the majority of specific IgE against Parietaria allergens, and, upon administration, both have an immunological behaviour in all alike that of the commercial extracts commonly used (11).

However, ParJ1 and ParJ2 do not constitute the sole ns-LTPs having allergenic properties. Recently, some scientific papers describing the characterisation of new allergenic molecules homologous to the ns-LTPs have been published (18-23).

Despite sequence heterogeneity, following cross-reactivity experiments between different ns-LTP allergens and related produced antibodies, it was demonstrated that ns-LTP constitute a widespread family of allergens (pan-allergen) as already described for profilin (19).

However, in comparison with the abundant information on the structure of this 'pan-allergen', exhaustive information on the localisation of the epitopes for IgE and IgG therein, as well as in the individual ns-LTP allergens (Parj1 and ParJ2 included) are not available (11, 12).

As a result of the mechanism in charge of the development of the allergic response, and of the verified role of IgG and IgE therein, the derivation of such a map would be instead of enormous relevance for the drafting of a novel therapeutic approach to these allergic forms (13).

In particular, the derivation of molecules with reduced or even absent IgE binding capability, yet concomitantly capable of inducing IgG response, and in particular of IgG4, might be a landmark both from a therapeutic and a preventive point of view.

Such a molecule would allow immunosuppression of the T cell response with reduced or even absent side effects.

In fact, to date the therapy of an undergoing allergy consists in the mere pharmacological cure of the allergic symptomatology.

A preventive therapy represented by the specific immunotherapy (SIT) actually consists in the subcutaneous administration of diluted quantities of allergen to the patient so as to suppress the specific reaction towards the allergen.

The majority of the commercial protein extracts used therefor however, are anyhow crude extracts, mixtures of several components in which a precise standardisation of the allergenic component is difficult.

Thus, the SIT strategy can entail the administration of allergenic components towards which the patient is not sensitive, inducing the secretion of IgEs specific towards other components of the extract. Moreover, the administration of the total allergen entails the possibility of side effects which, though with extremely low occurrence, could even cause anaphylactic shock.

Concerning in particular the Parietaria Judaica, epidemiological studies have also highlighted a different distribution of the two major allergens in the human population (12 millions of affected subjects in the Mediterranean area) where, approximately 20% of the PJ allergic patients do not exhibit a concomitant presence of IgE specific against both allergens. Therefore, an administration of total or partially purified crude extracts could entail an administration of major allergens to which the patient is not allergic.

Hence, the use of recombinant molecules, allowing a patient customized diagnosis and therapy, could represent a valid alternative to the traditional use of crude extracts.

In particular, the characterization and the development of alternative molecules with reduced side effects, i.e., having a reduced or absent interaction with the IgE while maintaining the capability of binding the IgGs (in particular the IgG4) with respect to the wild type and therefore the capability of immunosuppressing the T response, could allow to implement an alternative approach overcoming the disadvantages inherent to the traditional approach.

Such an alternative molecule with reduced anaphylactic capacity were in fact sought by producing crude formaldehyde- or glutaraldehyde- polymerised extracts (16).

Although effective, as demonstrated by clinical trials, these modified molecules have proved however to present the abovedescribed disadvantage of a difficult standardization of the extracts.

Following the' advent of genetic engineering both recombinant allergens immunologically similar to the native allergens (14 and 15), and recombinant allergen having instead a reduced allergenic activity with respect to the allergen wild type (therefore therapeutically suitable as a substitute of the latter), have been derived in a pure form.

None of such a mutant have not however been derived with particular reference to the ns-LTPs allergens.

### SUMMARY OF THE INVENTION

An object of the present invention is a variant of an allergen belonging to the ns-LTP protein family, specifically a hypoallergenic variant of a complete allergen or fragment thereof of the ns-LTD protein family.

In particular object of the invention is a variant of an allergen belonging to the ns-LTP family which lacks at least one of the four disulfide bridges constituting the structure of said allergen.

A first advantage of the variant of the present invention is that with respect to the native allergen it has a reduced or even absent capability of binding IgE, having concomitantly an intact capability of binding IgG of the said subjects.

This differential binding capability is particularly enhanced in the variants wherein such a missing bridge be localized in the amino-terminal region of the allergen at the domain alpha-helix 1- loop 1- alpha-helix 2, as they have a particularly reduced IgE binding activity, especially in the variants lacking at least two disulfide bridges.

In case such a variant be lacking three, or all four, of the disulfide bridges of the native allergen, the relevant IgE binding activity is reduced up to be substantially absent. Such a variant constitutes accordingly a preferred embodiment of the invention.

The relevance of such a differential binding capability of the variant of the invention lies in that according to the role of the two immunoglobulins in the molecular mechanism of the allergic response evidenced in the above paragraph, it turns out in molecules, that although immunogenic have a reduced or absent allergenicity.

Variants of the invention which mainly maintain most of amino acid sequence of the wild type allergen, and has accordingly substiantially the same length of the said allergen, constitute in this connection a preferred embodiment of the invention.

In particular variants consisting of a mutein in which at least one of the cysteines constituting said disulfide bridges is deleted, or substituted with an amino acid residue not capable of forming disulfide bridges, are preferred.

In this latter case, the substitution of the cysteine residue with serine or alanine, as amino acids tested compatible with the ns-LTP α-α-α-α-β structure, proved particularly effective.

The embodiment related to variants of the major allergens of Parietaria Judaica is particularly preferred.

In particular, the Parj1 variants, specifically the Parj1 muteins in which the deleted or substituted residues are the cysteines 4, 14, 29, 30, 50, 52, 75 and 91, and in particular the variants having a sequence selected in the group comprising the sequences reported in the sequence listing as SEQ ID NO: 4, SEQ ID NO: 6, SEQ ID NO: 8 and SEQ ID NO: 10, are especially relevant.

Object of the present invention is also a polynucleotide coding for the variants of the present invention, in particular for the above indicated muteins, and specifically the polynucleotides comprising a sequence selected in the group comprising the sequences reported in the sequence listing as SEQ ID NO: 3, SEQ ID NO: 5, SEQ ID NO: 7 and SEQ ID NO: 9, as well as the vectors comprising them.

In the light of what set forth above, object of the present invention are also any of the above mentioned variants for use as medicament or as a diagnostic agent, and in particular for use in the treatment and/or the prevention and/or the diagnosis of the allergic form associated with an allergen belonging to the family of ns-LTP proteins, (this in light of the pan-allergen characteristic verified for the ns-LTP allergens), in particular to the allergen corresponding to said variant.

In the specific case, hereto disclosed by way of example, of major allergens of Parietaria Judaica showing an uneven ability to stimulate serum IgE production in allergic patients, a specific diagnosis for the individual allergen may be attained using the variants of the invention, e.g., as follows: initially the recombinant version of each native molecule (parJ1 and Parj2) is used for a specific diagnosis of the allergy by skin prick test. Patients sensitive to one of two allergens can then be analyzed for positiveness to the individual allergen variants to which they tested positive in order to highlight negatively testing variants. Then, such variants can be administered in substitution of commercial protein extracts developing an allergen-specific immunotherapy. Further modes of employ for diagnostic as well as therapeutic ends are anyhow derivable by those skilled in the art in light of the knowledge of the state of the art.

Object of the present invention is also a pharmaceutical composition comprising a therapeutically effective quantity of at least one of the variants, or a polynucleotide or a vector among the above mentioned ones, and a pharmaceutically acceptable carrier, as well as all the matter compositions comprising at least one of the above mentioned molecules and one carrier chemically compatible therewith.

This pharmaceutically and/or chemically acceptable carrier can be any one carrier known to the art as suitable in pharmaceutical or matter compositions containing the molecules like the above mentioned ones, therefore in particular peptides and conjugates and/or oligonucleotides in any form, in particular in solid and in liquid form; an example of composition in liquid form is provided by compositions whose carrier is water, saline solutions, like, e.g., solutions containing NaCl and/or fosfate, or other solutions containing buffer molecules.

A still further object of the present invention is a kit for the derivation of a subject-customised allergogram, for an allergic form associated with an ns-LTP allergen comprising
- a first composition comprising said ns-LTP allergen in native form together with a chemically and/or pharmaceutically acceptable carrier;
- at least one composition comprising a single variant of said ns-LTP allergen as abovedescribed and a chemically and/or pharmaceutically acceptable carrier;
said allergogram being derivable contacting said compositions with immunoglobulins of said subject and observing the effects thus obtained.

Particularly preferred are the embodiments in which the kit comprises, besides said first composition, a number of compositions each comprising a single variant of said ns-LTP allergen, equal to the number of variants of said ns-LTPs allergen and that in which said allergen is a Parietaria Judaica allergen, specifically ParJ1 (in any one form thereof) or ParJ2 (in any one form thereof).

In particular such compositions can be contacted with immunoglobulins by 'skin prick test' in vivo, or on patient's tissues like, e.g., blood, in vitro. Other modes of employ of the kit of the present invention to diagnostic ends are derivable by those skilled in the art in light of the knowledge of the state of the art. The invention will be better described with the aid of the attached figures.

### DESCRIPTION OF THE FIGURES

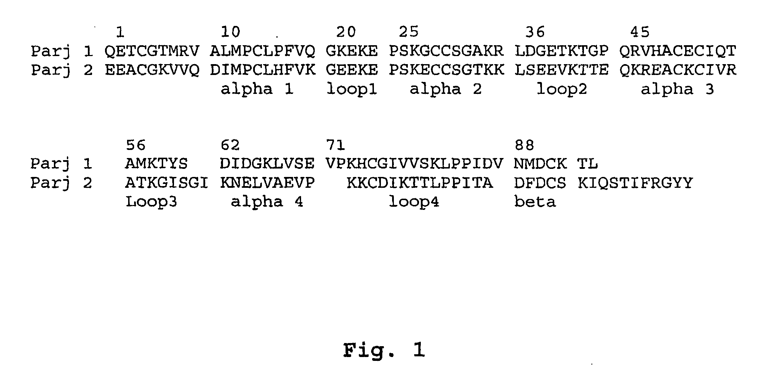

Fig. 1 reports the amino acid sequences of the native Par J 1.0102 and Par j 2.0101 aligned therebetween and with respect to the three-dimensional structure thereof. The notation of the amino acids relates to the sequence of the Par j 1.0102.

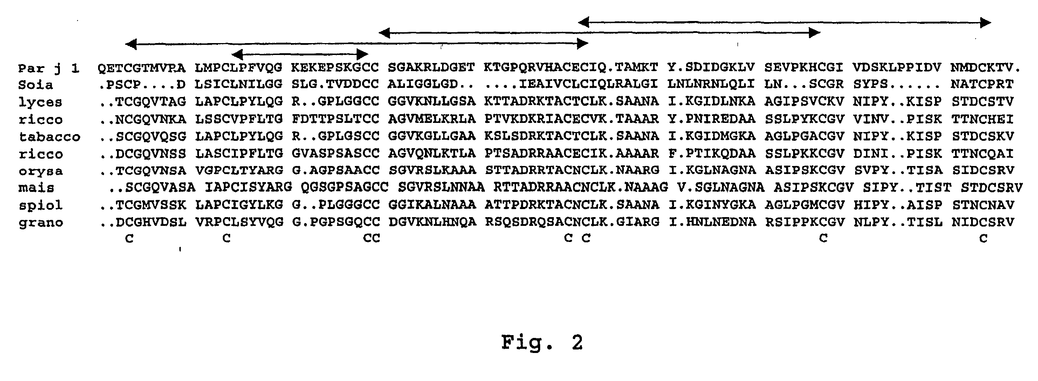

Fig. 2 shows the amino acid sequences of the Par j 1.0101 and of some ns-LTP proteins aligned thereamong. The arrows indicate the disulfide bridges present in the three-dimensional structure of the proteins. The amino acids are indicated in one-letter code. The Cs reported in the last row of the table indicate the cysteine residues conserved in all proteins of the ns-LTP family.

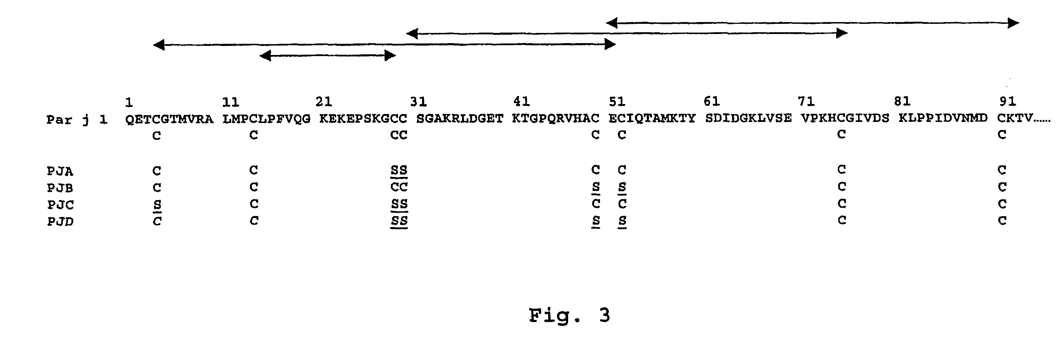

Fig. 3 reports the schematic representation of the mutants of the major allergen of the Parietaria Judaica Par j 1.0102, the sequence thereof being reported in the sequence listing as SEQ ID NO: 4, SEQ ID NO: 6, SEQ ID NO: 8 and SEQ ID NO: 10. The amino acids are reported in one-letter code. The underlined amino acids indicate the mutations effected in the native sequence. The arrows indicate the disulfide bridges.

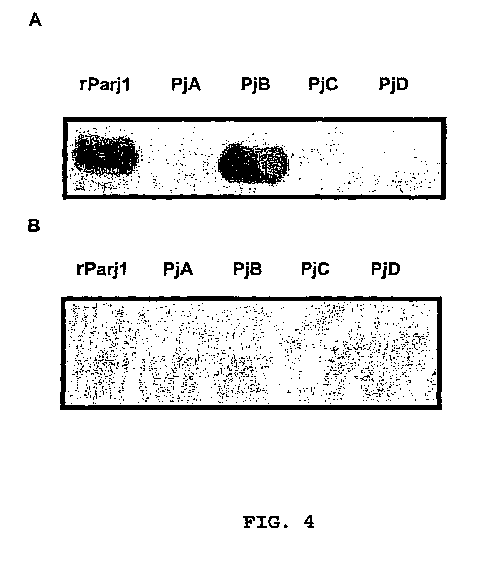

Fig. 4 reports in panel A the Western blot analysis results showing the IgE binding activity of the rParj1 and its disulfide bond variants by using a pool of sera (n=30) from monosensitive Pj allergic patients.

Panel B shows a Coomassie Brilliant Blue staining of the recombinant proteins used.

In both panels on the first lane the result referred to the native Par j 1.0101 is reported; on the second lane the result referred to the mutant PjA is reported; on the third lane the result referred to the mutant PjB is reported; on the fourth lane the result referred to the mutant PjC is reported; and on the fifth lane the result referred to the mutant PjD is reported.

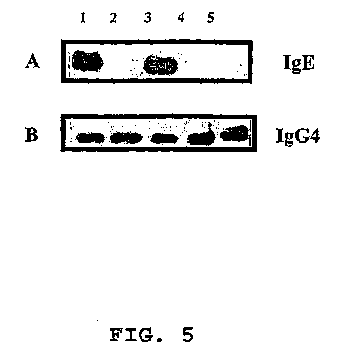

Fig. 5 shows in panel A the outcome of a Western Blot analysis on a pool of sera from PJ allergic patients, aimed at demonstrating the IgE binding capability (activity) of some mutants of the present invention extensively disclosed in example 3.

On the first lane the result referred to the native Par j 1.0101 is reported; on the second lane the result referred to the mutant PjA is reported; on the third lane the result referred to the mutant PjB is reported; on the fourth lane the result referred to the mutant PjC is reported; and on the fifth lane the result referred to the mutant PjD is reported.

In panel B the outcome of a Western blot analysis on a pool of sera from PJ allergic patients, aimed at demonstrating the IgG4 binding capability of the same abovedescribed mutants, it also extensively described in example 3, is shown.

On the first lane the result referred to the native Par j 1.0101 is reported; on the second lane the result referred to the mutant PjA is reported; on the third lane the result referred to the mutant PjB is reported; on the fourth lane the result referred to the mutant PjC is reported; and on the fifth lane the result referred to the mutant PjD is reported.

Fig. 6 reports an histogram showing the results of the ELISA detection experiment carried out using the pool of sera used in example 3, extensively described on the example 4. Black histogram indicate results obtained with allergic sera; dotted square indicated results obtained with non allergic serum. On the y axis the optical density measured and in the x axis the proteins tested (the rParj1 and its disulfide bond variants PjA, PjB, PjC, and PjD)) are reported.

Figure 7 shows ten diagram reporting the results of the ELISA detection carried out using monosensitive sera from ten Pj allergic patients extensively described on example 4, each diagram corresponding to a respective patient.

On each diagram on the y axis the optical density measured and in the x axis the proteins used (the rParj1 and its disulfide bond variants PjA, PjB, PjC, and PjD)) are reported.

On each diagram black squares indicate allergic sera, white squares a non allergic serum.

Fig. 8 reports an histogram showing the results of the ELISA detection of the Ig binding activity of a rabbit polyclonal immune and pre-immune antisera against rParj1, extensively described on the example 6.

On the y axis the optical density measured and in the x axis the antigens used (the rParj1 and its disulfide bond variants PjA, PjB, PjC, and PjD)) are reported.

Black squares indicate results obtained on rabbit immune, checkered squares results obtained on rabbit preimmune.

### DETAILED DESCRIPTION OF THE INVENTION

The experimental approach that led to the present invention consisted of a mutagenesis strategy aimed at the targeted disruption of all the disulfide bridges present in all the ns-LTPs. This in order to generate molecules with reduced or absent affinity to IgE, yet with intact affinity to the other classes of antibodies apt to compete with the specific IgEs against the native allergen. The ns-LTPs allergen ParJ1, whose sequence and structure is reported in comparison with Par J2 in figure 1, and in particular the isoform Par j 1.0102 (the primary sequence thereof being reported in the annexed sequence listing as SEQ ID NOs:1 and 2), was taken as a molecular model, to be used for carrying out mutagenesis and the subsequent verification of the properties of the obtained mutant. In particular, recombinant DNA technology was resorted to in a strategy of site-directed mutagenesis against cysteines 4, 29, 30, 50 and 52, i.e., the cysteines constituting the disulfide bridges according to the structural model known to the art.

The results of these experiments demonstrate the close relationship existing between the three-dimensional structure of the protein and the capability of forming epitopes for the IgEs, and in particular that the gradual disruption of the disulfide bridges causes a reduction of the serum IgEs binding activity thereof, whereas it does not affect the IgG4 binding activity thereof.

This in light of the Western blot analysis described in example 3, and in figure 5, in which lanes 2, 3 and 4 (see Fig. 5 panel A) show the reduction of the serum IgEs binding activity by the mutants of the present invention, which should be construed as three-dimensional mutants.

In particular, the Cys29-Cys30 mutant (PjA) shows a very weak binding band (Fig. 5 lane 2) whereas the Cys50-Cys52 mutant (PjB) is somehow still capable of binding the IgEs (Fig. 5, lane 3). Instead, more remarkable is the result shown by the PjC and PjD mutants (Fig. 5, lanes 4 and 5) for which no binding to human IgEs can be highlighted.

Such results have been confirmed by ELISA and IgE inhibition assays (see examples 4 and 5) where the PjB was the only variant still able to bind Parj1-specific IgE antibodies in solution while the other variants exhibited a very low inhibition capacity. The loss of additional disulfide bridges (PjC and PjD) leads to the absence of any IgE recognition (see Example 4 and Figure 4).

These results all together show that ns-LTP Parj1 variants lacking of at least one disulfide bridge have a reduced allergenicity, which is even absent in the variants wherein the lacking bridge is localized in the aminoterminal region of the allergen at the domain alpha-helix1-loop1-alphahelix2, in particular when the lacking bridges are at least two.

The maintenance by these variants of an overall antigenicity that, notwithstanding the reduced or absent allergenicity, is comparable or identical to the one of the wild type allergen, has been shown by experiments wherein binding activity of wild-type allergen and its variants to antibodies different than IgE, have been compared.

Such experiments are Western Blots carried out using as antibodies rabbit policlonal antibodies and IgG4 of Pj allergic patients, extensively described in examples 6 and 3 respectively.

In these experiments the hypoallergenic variants generated by genetic engineering presented a similar behavior compared to the wild type, with a low reduction of their binding activity towards the anti-rParj1 rabbit antibodies.

Accordingly, a variant that lacks of at least one disulfide bridges still contains several protein domains similar to the native molecule and, although at different extent, is apt to induce the production of IgG antibodies (see example 3 and 6). IgE production and/or IgE-mediated presentation of the allergen, would be prevented by such "blocking" antibodies and reducing T cell proliferation and release of cytokines (25).

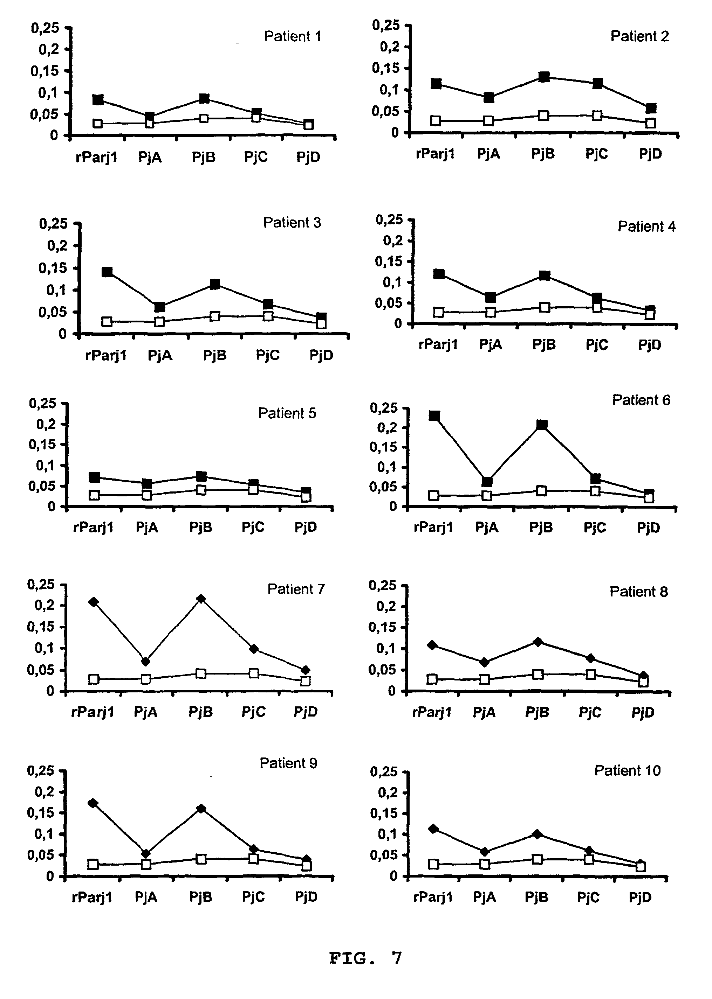

The above data have been confirmed also *in vivo* in particular by Skin Prick Test (SPT) analysis as described in example 7, where the pure recombinant proteins were tested on ten PJ allergic patients (the same analyzed by ELISA in example 4 and figure 7).

With regard to the single mutants, PjA showed a very low IgE binding activity and only 3 out of 10 patients with cutaneous Type I hypersensitivity and a reduced wheal area respect to that one induced by wild-type allergen. On the contrary, loss of the Cys50-Cys91 and Cys4-Cys52 bridges seems to have a minor effect since an IgE binding activity and a positive SPT are still present. The loss of additional disulfide bridges (PjC and PjD) leads to the absence of any cutaneous reaction (see example 7).

These results obtained analyzing individuals demonstrate the reliability of the above described data *in vitro*, and above all that while the disruption of the disulfide bridges in the amino terminal region (in the specific case Cys4-Cys52 and Cys50-Cys91) affect even if not markedly the human IgE binding capability of this mutant, the disruption of all the four bridges (in the specific case Cys4-Cys52, Cys14-Cys29, Cys30-Cys75 and Cys50-Cys91) have a devastating effect on the IgE recognition, and therefore on the allergenic response.

Accordingly, concerning the development of therapeutically useful hypoallergenic molecules, variants lacking of three or four bridges, as in the specific case PjC and PjD mutants, are considered as preferred embodiments. The above was demonstrated using a pool of sera as well as a cohort of individual patients, indicating that the obtained result is representative of the immune response of the allergic population.

It is pointed out that although obtained using specific variants derived by mutating the wild type allergen, these results are in fact not limited to the said specific variants, neither to the techniques used for the relevant derivation.

As such results are consequent to the modification of the three-dimensional structure of the allergen, they could anyhow have been obtained by any mutagenesis allowing the disruption of the disulfide bridges.

Accordingly any variant obtainable by deletion, substitution and/or the insertion of one or more amino acidic residue which results in variants lacking of at least one disulfide bridge is included in the object of the invention.

The strategy of point mutation has however the remarkable advantage of allowing the insertion of minimal variations at the level of the primary sequence of the native protein and therefore of generating mutants that are more likely not to interfere with the variant recognition operated by the T cells, and above all the possibility to generate proteins having a high reproducibility.

Variants having substantially the same length of the wild type are accordingly considered preferred.

With regard to the techniques used for deriving the variants of the invention, it is not limited to the genetic engineering ones, as they are obtainable by techniques like chemical mutagenesis (formaldhehyde and gluteraldhehyde) which allow the disruption of disulfide bridges even in absence of mutations.

The genetic mutagenesis imply however the remarkable advantage of allowing the generation of proteins having a high reproducibility, while the, chemical mutants do not ensure a denaturation pattern constant at every preparation.

Furthermore, as the strategy described herein is independent on the epitope sequence on itself since it is based on the modification of the three-dimensional structure of the IgE determinants, the adopted mutagenesis strategy is actually independent from the primary sequence of the allergen (and therefore from the sequence of the specific IgE epitopes).

For this reason variants of all the proteins with allergenic activity belonging to the ns-LTP family (including Parj2), are included in the object of the invention due to the conserved structure (cfr. e. g.s figure 2 wherein Par j1 sequence is reported in comparison with the sequence of other ns-LTPs together with the placement of the disulfides bridges).

In particular variant of the invention is not only any other mutein of the ParJ1 allergen or of other ns-LTP allergens which, independently from the mutation carried out (substitution and/or deletion of one or more amino acid residues) and of the way in which such a mutation is carried out (e.g., by the above mentioned techniques), retain a structure equivalent to that of the corresponding native allergen lacking at least one disulfide bridge.

Thus the disruption of the disulfide bridges in ns-LTP allergens underlies *per se* a limited or absent IgE binding ability of patients allergic to the related variants.

Moreover, in particular in light to what is known in the art concerning the high conservation of the structure and the cross-reactivity that have led to the singling out of the so-called ns-LTP pan-allergen (see above) these data are indicative not merely of a suitability in the therapy and prevention of the allergic forms caused by the allergens corresponding to the individual variants, but also in the therapy and prevention of allergic forms caused by ns-LTP allergens other than those corresponding to the variants used.

A person skilled in the art can derive on the basis of his knowledge any information suitable for deriving uses, compositions and kit described in the summary of the invention.

With the aid of the following examples, a more detailed description of specific embodiments will now be given, in order to give a better understanding of the objects, characteristics, advantages and operating methods of the present invention.

### EXAMPLES

### Example 1: Cloning and Expression of Par J 1.0102

For the production of the major allergen of Parietaria Judaica Par j 1.0102 the pQE30 prokaryotic vector (Qiagen) was used. The latter characteristically expresses recombinant proteins fused to a short histidine tail and inducible with isopropyl-β-D-thiogalactoside (IPTG). The histidine residues allow the purification of the recombinant protein by affinity chromatography.

For this reason, 1 ng of the P5 clone containing the processed version of the Par j 1.0102 (EMBL accession number X77414), the sequence thereof being reported in the annexed sequence listing as SEQ ID NO: 12, was subjected to 30 cycles of polymerase chain reaction (PCR) amplification at the following design: 94°C for 1 min, 52°C for 1 min, 72°C for 1 min. The synthetic primer oligonucleotides P5 forward and P5 reverse, the sequence thereof being reported in the annexed sequence listing as SEQ ID NO:11 and SEQ ID NO: 12, respectively, were used.

The fragment thus generated was fractionated on 1% agarose gel in I X TBE, extracted, purified and digested with Bam H1 and Hind III restriction enzymes and cloned in the Pqe30 VECTOR (Quiagen) previously digested with the same enzyme. The linearized vector and the digested fragments were incubated for 4 hours at 16° C in presence of the enzyme DNA ligase according to different stoichiometric ratios. The reaction mixture was then transformed in the bacterial strain M15. The recombinant clones were sequenced with the method of Sanger and the nucleotide sequence thus determined demonstrated that the DNA fragment inserted into the pQE30 vector was identical to that known in the art (10).

### Example 2: Cloning and expression of conformational mutants of ParJ 1.0102

PjA mutant (Cys29→Ser and Cys30→Ser) was generated using the Transformer Site-Directed Mutagenesis kit (Clontech) following the manufacturer's instruction and using the oligonucleotide P5 (29,30) reported in the sequence listing as SEQ ID NO: 13 (mapping from nucleotide 88 to nucleotide 105) and the Parj1 sequence as a template. PjB mutant (Cys50→Ser and Cys52→Ser) was generated by PCR using as primers the oligonucleotide P5(50-52)reported in the sequence listing as SEQ ID NO: 14 (mapping from nucleotide 91 to nucleotide 165) and P5 reverse oligonucleotide and 1 ng of the Parj1 clone as a template. The PCR fragment was digested with Pst I and Hind III restriction enzymes and ligated with the Pst I-Hind III linearized plasmid vector containing the Parj1 sequence (expressing the first 31 amino acids of the wild type Par j 1.0102 allergen). Pj C mutant (Cys4→Ser, Cys29→Ser and Cys30→Ser) was generated by PCR amplification using the PjA variant as a template. The cysteine residue at position 4 was mutated by PCR using the oligonucleotides P5(triple), the sequence thereof being reported as SEQ ID NO: 15, and P5 reverse.

After purification, PCR fragment was digested with Bam HI and Hind III enzymes and cloned in the pQE30 vector previously digested with the same restriction enzymes. PjD mutant (Cys29→Ser, Cys30→Ser, Cys50→Ser and Cys52→Ser) was generated using the Transformer Site-Directed Mutagenesis kit (Clontech) following the manufacturer's instruction and using the synthetic oligonucleotide P5 (29,30) reported in the sequence listing as SEQ ID NO: 13 and the PjB variant as a template. All clones were sequenced with the method of Sanger (24) and the mutations and the open reading frames confirmed (See Fig.3 for details).

With this process 4 independent mutants, hereinafter designated PjA (SEQ ID NO: 3 and SEQ ID NO: 4); PjB (SEQ ID NO: 5 and SEQ ID NO: 6), PjC (SEQ ID NO: 7 and SEQ ID NO: 8), and PjD (SEQ ID NO: 9 and SEQ ID NO: 10) were isolated.

### Example 3: Purification of recombinant proteins evaluation of the relevant capability of binding IgE of allergic patients

10 ml O/N culture of the recombinant clones (NM15 strain, Quiagen) were then used for an inoculation in 400 ml of 2YT broth (Bacto-tryptone 16 gr/l, Bacto-yeast 10 gr/l, NaCl 5 gr/l, pH 7,0) containing ampicillin and kanamycin at a final concentration of 100 µgr/ml and 10 µgr/ml, respectively.

A 1:40 dilution was grown for 1 hour at 37°C and, after that, induced with 1 mM isopropylthio-β-galactoside for 4 hours at 37° C. Cells were harvested by centrifugation and the recombinant proteins purified by using the His Trap kit (Pharmacia) following the manufacturer's instructions. Recombinant proteins, binding the HiTrap chelating column, were eluted using a buffer containing: 20mM phosphate buffer pH7.4. 0,5 M NaCl, 8 M UREA and 500 mM imidazole; fractions were analysed by 16% SDS-PAGE and Coomassie Brilliant Blue staining. Fractions containing the purified protein were then diluted 1:100 in a buffer containing 20mM phosphate buffer pH7.4. 0,5 M NaCl and 20 mM imidazole to allow refolding of the protein, reloaded on the His Trap column and eluted with a buffer with no denaturing agents (20mM phosphate buffer pH7.4. 0,5 M NaCl and 500 mM imidazole). Recombinant proteins were then desalted using a centrifugal filter device (Centriprep, Millipore) and analysed for their capability of binding human IgE from Pj allergic patients by Western blot as previously described (12), using a pool of sera (n=30) of Pj allergic patients which did not receive any specific immunotherapy.

This analysis showed that the PjB mutant was still capable of binding human IgE while the PjA mutant retains only a weak IgE binding activity. The PjC and PjD mutants did not show any IgE binding activity suggesting that the IgE recognition was dependent on the three-dimensional folding of the protein (Fig.4 Panel A).

After that, membranes were stripped and reprobed with a His-tag specific reagent (INDIA™ Hisprobe-HRP, Pierce,USA) to check that the IgE-allergen complex was specific for the recombinant fused proteins. The concentration of the recombinant proteins was determined by densitometric analysis of SDS-PAGE gels stained with Coomassie Brilliant Blue (see figure 4 panel B).

As a confirmation of this experiment another Western blot carried out using IgE and IgG4 of allergic patients.

Then, the proteins purified were fractionated on 16% PAGE-SDS and transferred on nitro-cellulose thanks to a Dry-blot system (Millipore). The membrane was incubated for 12-14 hours with a pool of sera from Pj allergic patients (1:5 dilution) in PBS-tween. The protein-human IgE and IgG4 binding complexes are highlighted using respectively a secondary anti-IgE and anti-IgG4 antibodies conjugated to radish peroxidase. Thus, the complexes are highlighted using a chemioluminescence system (Super-signal, Pierce). The relevant results are reported in figure 4.

### Example 4: Elisa detection

The same pool of allergic sera from non-sensitive PJ allergic patients used in example 3 has been used in an ELISA experiment, showing the IgE binding activity of the rParj1 and its disulfide bond variants. A non allergic subject has been tested as a negative control on the ELISA.

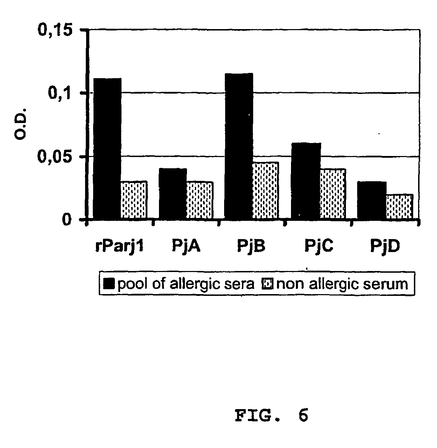

The results confirm the pattern of reaction of the experiment of the example 3 (Fig.4 Panel A) with the PjB variant reacting in a way comparable to the wild-type allergen. A non allergic serum is shown as a negative control (Fig.6).

The IgE binding activity of the four Parj1 disulfide bond variants was also tested by ELISA using sera from ten monosensitive Pj allergic patients. Analysis of single sera showed a remarkable homogeneity of the reaction. In particular, the Cys4-Cys52 and Cys50-Cys91 bridges did not influence the allergenicity of the protein since this mutant (PjB) showed an IgE binding activity comparable to the wild-type allergen. On the other hand, the Cys14-Cys29 and Cys30-Cys75 bridges seem to be crucial for the IgE recognition. All the variants lacking those two bonds (PjA, PjC and PjD) presented low or even absent IgE binding activity. (Fig.7)

ELISA detection has been performed by adding 200 µl of a solution containing 5 µg/ml of antigen in coating buffer (sodium carbonate buffer pH 9,5) to each well of polystyrene plates overnight at room temperature. After several washing steps (1XPBS, 0.1% Tween 20) plates were saturated with a solution containing 5% BSA, 0,5% Tween 20 in coating buffer. After washing, 200 µl of serum (1:5 dilution) from Pj allergic patients or from a non allergic subject were incubated for 4 hours at room temperature. Bound IgE antibodies were detected with a goat antihuman IgE-HRP conjugate (Biosource International) diluted at a concentration of 0,5 ng/ml in 1XPBS, 0,25% BSA, 0,1% Tween 20 for 1 hour at room temperature. After several washes, colorimetric reaction was developed by adding 0,2 ml/well of substrate solution (0,4 mg/ml o-phenylendiamine in 0,1 M citrate buffer). Optical density was read at 495 nm in a BIO-RAD microplate reader.

### Example 5: IgE inhibition assay

In order to investigate whether the disulfide bond variants were able to inhibit the binding of the IgE to the rParj1, increasing amount of each recombinant mutant were incubated with a pool of sera (n=10) of Pj monosensitive allergic patients.

The ability of the Parj1 disulfide variants to interact with IgE antibodies was determined by an ELISA inhibition experiment. A pool of sera (1:5 dilution) from ten monosensitive Pj allergic patients was preincubated overnight with increasing concentration of each disulfide bond variant (0,25-20 µg/ml of protein). The solutions were added to the ELISA wells coated with 5mg/ml of rParj1 and the ELISA steps were performed as above described. Percentage of inhibition was calculated according to the formula: %= 100-OD_{A}/OD_{B}X100, where OD_{A} and OD_{B} represent the optical density read with the inhibited and non-inhibited pool of sera respectively.

The results are reported in the following Table I

**Table I:**

| Inhibition of IgE binding | |
|---|---|
| Protein tested | %inhibition |
| rParj1 | 95% |
| PjA | 16% |
| PjB | 85% |
| PjC | 14% |
| PjD | 15% |

The data reported in Table I suggests that all the variants lacking, at least, Cys14-Cys29 and Cys30-Cys75 disulfide bonds exhibit a comparable low level of inhibition (about 15%). On the contrary, the PjB variant (Cys50→Ser and Cys52→Ser) showed a high percentage of inhibition retaining a substantial ability of binding human IgE (about 85%).

### Example 6: Rabbit policlonal binding activity

Rabbits were immunized by PRIMM sr1 (Milan, Italy) using the rParj1 allergen. As a control, rabbit polyclonal antibodies were analysed on a Western blot using a *Parietaria judaica* crude extract detecting a band of about 14000 Da corresponding to Parj1 native molecular weight. ELISA plates were coated at the same conditions as above described, with the wild-type Parj1 and with equal amount of each recombinant disulfide bond variant, were probed with an anti-rParj1 specific polyclonal serum to analyse their binding activity.

Rabbit preimmune and immune sera were diluted at a concentration of 6 ng/ml and 200 µl of these solutions were incubated at room temperature for 1 hour. Wells were washed three times in 1XPBS, 0,1% Tween 20 and bound antibodies were detected using a donkey antirabbit Ig HRP linked (Amersham) at a 1:1000 dilution. Colorimetric reaction and optical density were performed as above described.

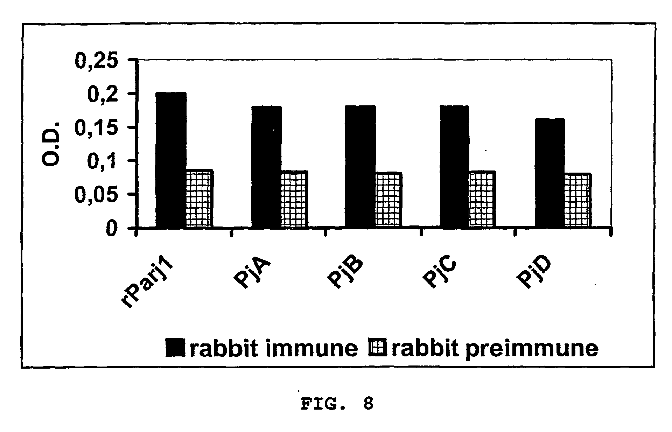

The data obtained suggest that the PjA, PjB and PjC variants show a similar behavior exhibiting a slight reduction of their binding ability (about 10%) compared to the Parj1 binding. The PjD variant showed a reduced binding activity (about 20%) while the preimmune serum did not show any reactivity towards the proteins (Fig.8).

### Example 7: Skin Prick test experiments with purified muteins

Ten patients, with a clear history of *Parietaria judaica* allergy and with skin prick test (SPT) monosensitivity to Pj commercial extract, were analysed in this study. All the patients did not receive immunotherapy against Pj pollen and were not receiving glucocorticosteroid treatment. Allergens were used at 1µg/ml concentration diluted in 0,9% NaCl. About 20 µl of the test solution was placed on the forearms at a distance of more than 2.5 cm between each prick. All tests were performed in duplicate. Histamine was used as positive control and 0,9% NaCl solution as a negative control. Reactions were measured after 20 min. By comparison with the wheal area generated by histamine (100%), positive SPT were divided in three classes: 4+ were assigned to SPT with an area ≥ 100% of area induced by histamine; 3+ were assigned to an area ≥80-100% and 2+ to an area ≥ 50-80%. Two non-allergic patients (P.C. and D.G.) were tested as negative controls. Each subject was informed by the investigators and signed informed consent before the test.

All patients showed a positive cutaneous reaction to the rParj1 allergen. PjB was capable of inducing Type I immediate hypersensitivity in 9 out of 10 of the tested patients. PjA gave positive reaction in 3 out of 10 of the patients and the wheal areas induced by prick were reduced in size respect to that ones triggered by the wild-type allergen. The PjC and PjD did not give any SPT reaction. None reactions have been observed when non allergic subjects were tested as reported in the following table II.

**Table II:**

| Skin prick test of the rPar J and its disulfide bond variants | | | | | |
|---|---|---|---|---|---|
| Patient No. | RParj1 | PjA | PjB | PjC | PjD |
| 1 | ++++ | - | - | - | - |
| 2 | +++ | ++ | +++ | - | - |
| 3 | ++++ | - | +++ | - | - |
| 4 | ++++ | - | ++++ | - | - |
| 5 | ++++ | ++ | +++ | - | - |
| 6 | ++++ | - | +++ | - | - |
| 7 | ++++ | - | ++++ | - | - |
| 8 | ++++ | ++ | +++ | - | - |
| 9 | +++ | - | ++ | - | - |
| 10 | +++ | - | ++ | - | - |
| P.C. | - | - | - | - | - |
| D.G. | - | - | - | - | - |

### REFERENCES

1. Menegozzo, M., D. Geraci, and A. Ruffilli, Isolation and characterization of an allergenic fraction from Parietaria officinalis pollen. Immunochemistry, 1976. 13 (5) : p. 475-6.
2. Geraci, D., U. Oreste, and A. Ruffilli, Purification and characterization of allergens from Parietaria officinalis pollen. Immunochemistry, 1978. 15 (7) : p. 491-8.
3. Geraci, D., et al., Immunochemical characterization of antigens of Parietaria Judaica pollen. Identification of allergens by means of crossed radio immunoelectrophoresis. Int Arch Allergy Appl Immunol, 1985. 78(4): p. 421-8.
4. Corbi, A.L. and J. Carreira, Identification and characterization of Parietaria Judaica allergens. Int Arch Allergy Appl Immunol, 1984. 74(4): p. 318-23.
5. Corbi, A.L., et al., Isolation of the major IgE-binding protein from Parietaria Judaica pollen using monoclonal antibodies. Mol Immunol, 1985. 22(9): p. 1081-9.
6. Corbi, A.L., R. Ayuso, and J. Carreira, Identification of IgE binding polypeptides cross-reactive with the Parietaria Judaica main allergenic polypeptide. Mol Immunol, 1986. 23(12): p. 1357-63.
7. Ford, S.A., et al., Identification of Parietaria Judaica pollen allergens. Int Arch Allergy Appl Immunol, 1986. 79(2): p. 120-6.
8. Ayuso, R., F. Polo, and J. Carreira, Purification of Par j I, the major allergen of Parietaria Judaica pollen. Mol Immunol, 1988. 25(1): p. 49-56.
9. Cocchiara, R., et al., Purification of ParJ I, a major allergen from Parietaria, Judaica pollen. Int Arch Allergy Appl Immunol, 1989. 90(1): p. 84-90.
10. Duro, G., et al., Isolation and characterization of two cDNA clones coding for isoforms of the Parietaria Judaica major allergen Par j 1.0101. Int Arch Allergy Immunol, 1997. 112(4): p. 348-55.
11. Duro, G., et al., cDNA cloning, sequence analysis and allergological characterization of Par j 2.0101, a new major allergen of the Parietaria Judaica pollen. FEBS Lett, 1996. 399(3): p. 295-8.
12. Colombo, P., et al., Identification of an immunodominant IgE epitope of the Parietaria Judaica major allergen. J Immunol, 1998. 160(6): p. 2780-5.
13. Rolland, J. and R. O'Hehir, Immunotherapy of allergy: anergy, deletion, and immune deviation. Curr Opin Immunol, 1998. 10(6): p. 640-5.
14. Singh, M.B., N. de Weerd, and P.L. Bhalla, Genetically engineered plant allergens with reduced anaphylactic activity. Int Arch Allergy Immunol, 1999. 119(2): p. 75-85.
15. Valenta, R., et al., Genetically engineered and synthetic allergen derivatives: candidates for vaccination against type I allergy. Biol Chem, 1999. 380(7-8): p. 815-24.
16. Maasch, H.J. and D.G. Marsh, Standardized extracts modified allergens--allergoids. Clin Rev Allergy, 1987. **5**(1): p. 89-106.
17. Poznanski, J., et al., Solution structure of a lipid transfer protein extracted from rice seeds. Comparison with homologous proteins. Eur J Biochem, 1999. 259(3): p. 692-708.
18. Asero, R., Detection and clinical characterization of patients with oral allergy syndrome caused by stable allergens in Rosaceae and nuts. Ann Allergy Asthma Immunol, 1999. 83(5): p. 377-83.
19. Asero, R., et al., Lipid Transfer Protein: A Pan-Allergen in Plant-Derived Foods That Is Highly Resistant to Pepsin Digestion. Int Arch Allergy Immunol, 2000. 122(1): p. 20-32.
20. Pastorello, E.A., et al., Complete amino acid sequence determination of the major allergen of peach (Prunus persica) Pru p 1. Biol Chem, 1999. 380(11): p. 1315-20.
21. Pastorello, E.A., et al., Evidence for a lipid transfer protein as the major allergen of apricot. J Allergy Clin Immunol, 2000. 105(2 Pt 1): p. 371-7.
22. Sanchez-Monge, R., et al., Lipid-transfer proteins are relevant allergens in fruit allergy. J Allergy Clin Immunol, 1999. 103(3 Pt 1): p. 514-9.
23. Toriyama, K., et al., Molecular cloning of a cDNA encoding a pollen extracellular protein as a potential source of a pollen allergen in Brassica rapa. FEBS Lett, 1998. 424(3): p. 234-8.
24. Sanger F, Nicklen S, Coulson AR: DNA sequencing with chain-terminating inhibitors. Proc Natl Acad Sci U S A 1977;74: 5463-7.
25. Akdis CA, Blaser K: Mechanisms of allergen-specific immunotherapy. Allergy 2000;55: 522-30.
26. Briner TJ, Kuo MC, Keating KM, Rogers BL, Greenstein JL: Peripheral T-cell tolerance induced in naive and primed mice by subcutaneous injection of peptides from the major cat allergen Fel d I. Proc Natl Acad Sci U S A 1993;90: 7608-12.
27. Muller U, Akdis CA, Fricker M, Akdis M, Blesken T, Bettens F, Blaser K: Successful immunotherapy with T-cell epitope peptides of bee venom phospholipase A2 induces specific T-cell anergy in patients allergic to bee venom. J Allergy Clin Immunol 1998;101: 747-54.
28. Pastorello EA, Ortolani C, Baroglio C, Pravettoni V, Ispano M, Giuffrida MG, Fortunato D, Farioli L, Monza M, Napolitano L, Sacco M, Scibola E, Conti A: Complete amino acid sequence determination of the major allergen of peach (Prunus persica) Pru p 1. Biol Chem 1999;380: 1315-20.
29. Shin, D. H.,Lee, J. Y.,Hwang, K. Y.,Kim, K. K.,Suh, S. W. "High-resolution crystal structure of the non-specific lipid-transfer protein from maize seedlings" in Structure, 1995, 3,(2), p. 189-199

## Claims

1. A variant of an allergen belonging to the family of the ns-LTP proteins, said variant maintaining most of the amino acid sequence of the wild type allergen, said variant lacking at least one of the four disulfide bridges constituting the structure of said allergen and exhibiting reduced IgE binding capability while maintaining the capability of inducing IgG response.

2. The variant according to claim 1, said variant lacking at least one disulfide bridge in the amino-terminal region of said allergen.

3. The variant according to claim 1 or 2, said variant lacking two of said disulfide bridges constituting the structure of said allergen.

4. The variant according to claim 1, said variant lacking three, or four of said disulfide bridges constituting the structure of said allergen.

5. The variant according to any one of the claims 1 to 4, said variant being a mutein wherein at least one of the cysteines constituting said disulfide bridges is deleted.

6. The variant according to any one of the claims 1 to 5, said variant being a mutein wherein at least one of the cysteines constituting said disulfide bridges is substituted with an amino acid residue not capable of forming disulfide bridges.

7. The variant according to claim 6, wherein said amino acid residue is serine or alanine.

8. The variant according to any of claims 1 to 7, wherein said allergen is one of the major allergens of Parietaria Judaica.

9. The variant according to claim 8, wherein said allergen is Parj1, and said cysteines constituting the disulfide bridges are the cysteines 4, 14, 29, 30, 50, 52, 75 and 91.

10. The variant according to claim 9, wherein the sequence of said variant comprises a sequence selected from the group consisting of the sequences reported in the sequence listing as SEQ ID NO: 1, SEQ ID NO: 3, SEQ ID NO: 5 and SEQ ID NO: 7.

11. The variant according to any one of the claims 1 to 10 for use as a medicament or as a diagnostic agent.

12. The variant according to claim 11 for use in a patient customised treatment and/or prevention of allergic form associated with an allergen belonging to the family of the ns-LTP proteins.

13. The variant according to claim 12 which is a variant of a major allergen of Parietaria Judaica and wherein the allergic form is associated with an allergen of Parietaria Judaica.

14. The variant according to claims 12 or 13 for use in the treatment of itch, erythema, edema, wheal, rash (urticaria) formation, rhino-conjunctivitis (seasonal allergies), bronchoconstriction, asthma and anaphylaxis.

15. The variant according to claims 11 to 14 for use in preventive specific immunotherapy.

16. The variant according to claim 11 for use as diagnostic agent for patient customised diagnosis of allergic forms.

17. A polynucleotide coding for the variant according to any one of the claims 1 to 11.

18. The polynucleotide according to claim 17, wherein said sequence of said variant comprises a sequence selected in the group comprising the sequences reported in the sequence listing as SEQ ID NO: 2, SEQ ID NO: 4, SEQ ID NO: 6 and SEQ ID NO: 8.

19. A vector comprising at least one polynucleotide according to claim 17 or 18.

20. An use of a variant according to any one of the claims 1 to 11, for the preparation of a medicament for the treatment and/or the prevention of allergic form associated with an allergen belonging to the family of the ns-LTP proteins.

21. The use according to claim 20, wherein said allergen belonging to the family of ns-LTP proteins is the allergen corresponding to said variant.

22. The use according to claim 20 or 21, wherein said allergen is a major allergen of Parietaria Judaica and said allergic form associated with said allergen is an allergic form from Parietaria Judaica.

23. A pharmaceutical composition comprising at least one variant according to any one of the claims 1 to 11, and a pharmaceutically acceptable carrier.

24. A pharmaceutical composition comprising at least one polynucleotide according to claim 17 or 18, and/or one vector according to claim 19, and a pharmaceutically acceptable carrier.

25. A diagnostic agent comprising at least one variant according to any one of the claims 1 to 11, and a pharmaceutically acceptable carrier.

26. A kit for the derivation of a subject-customised allergogram for an allergic form associated with an ns-LTP allergen, comprising
- a first composition comprising said ns-LTP allergen in native form together with an acceptable carrier;
- at least one composition comprising a single variant of said ns-LTP allergen according to any one of the claims 1 to 11 and an acceptable carrier;
said allergogram being derivable contacting said compositions with immunoglobulins of said subject and observing the effects thus obtained.

27. The kit according to claim 26, comprising said first composition and a number of compositions each comprising a single variant of said ns-LTP allergen, equal to the number of the variants of said ns-LTP allergen.

28. The kit according to claim 26 or 27, wherein said ns-LTP allergen is ParJ1.

## Patentansprüche

1. Variante eines Allergens, welches zu der Familie der ns-LTP-Proteine gehört, wobei die genannte Variante den größten Teil der Aminosäuresequenz des Wildtyp-Allergens aufweist, der genannten Variante wenigstens eine der vier Disulfid-Brücken, welche die Struktur des genannten Allergens erzeugt, fehlt und die genannte Variante eine reduzierte IgE-Bindungsfähigkeit aufweist, während sie die Fähigkeit zur Induzierung einer IgE-Antwort beibehält.

2. Die Variante gemäß Anspruch 1, wobei der genannten Variante wenigstens eine Disulfid-Brücke in dem aminoterminalen Bereich des genannten Allergens fehlt.

3. Die Variante gemäß Anspruch 1 oder 2, wobei der genannten Variante zwei der genannten Disulfid-Brücken, welche die Struktur des genannten Allergens erzeugen, fehlen.

4. Die Variante gemäß Anspruch 1, wobei der genannten Variante drei oder vier der genannten Disulfid-Brücken, welche die Struktur des genannten Allergens erzeugen, fehlen.

5. Die Variante gemäß einem der Ansprüche 1 bis 4, wobei die genannte Variante ein Mutein ist, worin wenigstens eines der Cysteine, welche die genannten Disulfid-Brücken bilden, deletiert ist.

6. Die Variante gemäß einem der Ansprüche 1 bis 5, wobei die genannte Variante ein Mutein ist, worin wenigstens eines der Cysteine, welche die genannten Disulfid-Brücken bilden, mit einem Aminosäurerest substituiert ist, welcher nicht fähig ist, die Disulfid-Brücken auszubilden.

7. Die Variante gemäß Anspruch 6, worin der genannte Aminosäurerest Serin oder Alanin ist.

8. Die Variante gemäß einem der Ansprüche 1 bis 7, worin das genannte Allergen eines der Hauptallergene von Parietaria Judaica ist.

9. Die Variante gemäß Anspruch 8, worin das genannte Allergen Parj1 ist und die genannten Cysteine, welche die Disulfid-Brücken erzeugen, die Cysteine 4, 14, 29, 30, 50, 52, 75 und 91 sind.

10. Die Variante gemäß Anspruch 9, worin die Sequenz der genannten Variante eine Sequenz umfasst, welche aus der Gruppe bestehend aus den Sequenzen, die in dem Sequenzprotokoll als SEQ ID NO: 1, SEQ ID NO: 3, SEQ ID NO: 5 und SEQ ID NO: 7 dargestellt sind, ausgewählt ist.

11. Die Variante gemäß einem der Ansprüche 1 bis 10 für die Verwendung als ein Medikament oder als ein Diagnosemittel.

12. Die Variante gemäß Anspruch 11 für die Verwendung in einer Patientenspezifischen Behandlung und/oder Prävention einer Allergieform, welche mit einem Allergen assoziiert ist, das zu der Familie der ns-LTP-Proteine gehört.

13. Die Variante gemäß Anspruch 12, welche eine Variante eines Hauptallergens von Parietaria Judaica ist und worin die Allergieform mit einem Allergen von Parietaria Judaica assoziiert ist.

14. Die Variante gemäß den Ansprüchen 12 oder 13 für die Verwendung in der Behandlung von Juckreiz, Erythemen, Ödemen, Quaddeln, Ausschlag(Urticaria)-Bildung, Rhinoconjunctivitis (saisonale Allergien), Bronchokonstriktion, Asthma und Anaphylaxie.

15. Die Variante gemäß den Ansprüchen 11 bis 14 für die Verwendung in der präventiven spezifischen Immuntherapie.

16. Die Variante gemäß Anspruch 11 für die Verwendung als Diagnosemittel für die Patienten-spezifische Diagnose von Allergieformen.

17. Polynucleotid, welches für die Variante gemäß einem der Ansprüche 1 bis 11 codiert.

18. Das Polynucleotid gemäß Anspruch 17, worin die genannte Sequenz der genannten Variante eine Sequenz umfasst, welche aus der Gruppe umfassend die Sequenzen, die in dem Sequenzprotokoll als SEQ ID NO: 2, SEQ ID NO: 4, SEQ ID NO: 6 und SEQ ID NO: 8 dargestellt sind, ausgewählt ist.

19. Vektor, der wenigstens ein Polynucleotid gemäß den Ansprüchen 17 oder 18 u m-fasst.

20. Verwendung einer Variante gemäß einem der Ansprüche 1 bis 11 für die Herstellung eines Medikaments für die Behandlung und/oder Prävention von einer Allergieform, welche mit einem Allergen assoziiert ist, das zu der Familie der ns-LTP-Proteine gehört.

21. Die Verwendung gemäß Anspruch 20, worin das zu der Familie der ns-LTP-Proteine gehörende genannte Allergen das Allergen ist, welches der genannten Variante entspricht.

22. Die Verwendung gemäß Anspruch 20 oder 21, worin das genannte Allergen ein Hauptallergen von Parietaria Judaica ist und die genannte Allergieform, welche mit dem genannten Allergen assoziiert ist, eine Allergieform von Parietaria Judaica ist.

23. Pharmazeutische Zusammensetzung, welche wenigstens eine Variante gemäß einem der Ansprüche 1 bis 11 und einen pharmazeutisch annehmbaren Träger umfasst.

24. Pharmazeutische Zusammensetzung, welche wenigstens ein Polynucleotid gemäß Anspruch 17 oder 18 und/oder einen Vektor gemäß Anspruch 19 und einen pharmazeutisch annehmbaren Träger umfasst.

25. Diagnosemittel, welches wenigstens eine Variante gemäß einem der Ansprüche 1 bis 11 und einen pharmazeutisch annehmbaren Träger umfasst.

26. Kit für das Erhalten eines Personen-spezifischen Allergogramms für eine Allergieform, welche mit einem ns-LTP-Allergen assoziiert ist, umfassend
- eine erste Zusammensetzung, welche das genannte ns-LTP-Allergen in nativer Form zusammen mit einem annehmbaren Träger umfasst;
- wenigstens eine Zusammensetzung, welche eine einzige Variante des genannten ns-LTP-Allergens gemäß einem der Ansprüche 1 bis 11 und einen anneh m-baren Träger umfasst;
wobei das genannte Allergogram durch Inkontaktbringen der genannten Zusammensetzungen mit Immunglobulinen der genannten Person und durch Beobachten der Wirkungen, welche dadurch erhalten werden, ableitbar ist.

27. Das Kit gemäß Anspruch 26, welches die genannte erste Zusammensetzung und eine Anzahl von Zusammensetzungen, die jeweils eine einzige Variante des genannten ns-LTP-Allergens umfassen und die der Anzahl der Varianten des genannten ns-LTP-Allergens entspricht, umfasst.

28. Das Kit gemäß Anspruch 26 oder 27, worin das genannte ns-LTP-Allergen ParJ1 ist.

## Revendications

1. Variant d'un allergène appartenant à la famille des protéines ns-LTP, ledit variant conservant la plus grande partie de la séquence d'aminoacides de l'allergène de type sauvage, ledit variant étant dépourvu d'au moins l'un des quatre ponts disulfure constituant la structure dudit allergène et présentant une capacité de liaison d'IgE réduite tout en conservant la capacité d'induire une réponse IgG.

2. Variant selon la revendication 1, ledit variant étant dépourvu d'au moins un pont disulfure dans la région amino-terminale dudit allergène.

3. Variant selon la revendication 1 ou 2, ledit variant étant dépourvu de deux desdits ponts disulfure constituant la structure dudit allergène.

4. Variant selon la revendication 1, ledit variant étant dépourvu de trois ou quatre desdits ponts disulfure constituant la structure dudit allergène.

5. Variant selon l'une quelconque des revendications 1 à 4, ledit variant étant une mutéine où au moins l'une des cystéines constituant lesdits ponts disulfure est délétée.

6. Variant selon l'une quelconque des revendications 1 à 5, ledit variant étant une mutéine où au moins l'une des cystéines constituant lesdits ponts disulfure est remplacée par un résidu d'aminoacide incapable de former des ponts disulfure.

7. Variant selon la revendication 6 où ledit résidu d'aminoacide est la sérine ou l'alanine.

8. Variant selon l'une quelconque des revendications 1 à 7 où ledit allergène est l'un des allergènes majeurs de Parietaria Judaica.

9. Variant selon la revendication 8 où ledit allergène est Parj1, et lesdites cystéines constituant les ponts disulfure sont les cystéines 4, 14, 29, 30, 50, 52, 75 et 91.

10. Variant selon la revendication 9 où la séquence dudit variant comprend une séquence choisie dans le groupe consistant en les séquences présentées dans le listage de séquences comme SEQ ID NO : 1, SEQ ID NO : 3, SEQ ID NO : 5 et SEQ ID NO : 7.

11. Variant selon l'une quelconque des revendications 1 à 10 destiné à être utilisé comme médicament ou comme agent de diagnostic.

12. Variant selon la revendication 11 destiné à être utilisé dans un traitement adapté au patient et/ou la prévention d'une forme allergique associée avec un allergène appartenant à la famille des protéines ns-LTP.

13. Variant selon la revendication 12 qui est un variant d'un allergène majeur de Parietaria Judaica et où la forme allergique est associée avec un allergène de Parietaria Judaica.

14. Variant selon les revendications 12 ou 13 destiné à être utilisé dans le traitement du prurit, de l'érythème, de l'oedème, de la boule d'oedème, de la formation d'éruption (urticaire), de la rhino-conjonctivite (allergies saisonnières), de la bronchoconstriction, de l'asthme et de l'anaphylaxie.

15. Variant selon les revendications 11 à 14 destiné à être utilisé dans l'immunothérapie spécifique préventive.

16. Variant selon la revendication 11 destiné à être utilisé comme agent de diagnostic pour un diagnostic de formes allergiques adapté au patient.

17. Polynucléotide codant le variant selon l'une quelconque des revendications 1 à 11.

18. Polynucléotide selon la revendication 17 où ladite séquence dudit variant comprend une séquence choisie dans le groupe comprenant les séquences présentées dans le listage de séquences comme SEQ ID NO : 2, SEQ ID NO : 4, SEQ ID NO : 6 et SEQ ID NO : 8.

19. Vecteur comprenant au moins un polynucléotide selon la revendication 17 ou 18.

20. Utilisation d'un variant selon l'une quelconque des revendications 1 à 11 pour la préparation d'un médicament pour le traitement et/ou la prévention d'une forme allergique associée avec un allergène appartenant à la famille des protéines ns-LTP.

21. Utilisation selon la revendication 20 où ledit allergène appartenant à la famille des protéines ns-LTP est l'allergène correspondant audit variant.

22. Utilisation selon la revendication 20 ou 21 où ledit allergène est un allergène majeur de Parietaria Judaica et ladite forme allergique associée avec ledit allergène est une forme allergique de Parietaria Judaica.

23. Composition pharmaceutique comprenant au moins un variant selon l'une quelconque des revendications 1 à 11 et un support pharmaceutiquement acceptable.

24. Composition pharmaceutique comprenant au moins un polynucléotide selon la revendication 17 ou 18 et/ou un vecteur selon la revendication 19, et un support pharmaceutiquement acceptable.

25. Agent de diagnostic comprenant au moins un variant selon l'une quelconque des revendications 1 à 11 et un support pharmaceutiquement acceptable.

26. Kit pour l'obtention d'un allergogramme adapté au sujet pour une forme allergique associée avec un allergène ns-LTP, comprenant
- une première composition comprenant ledit allergène ns-LTP sous forme native ainsi qu'un support acceptable ;
- au moins une composition comprenant un seul variant dudit allergène ns-LTP selon l'une quelconque des revendications 1 à 11 et un support acceptable ;
ledit allergogramme pouvant être obtenu par mise en contact desdites compositions avec des immunoglobulines dudit sujet et observation des effets ainsi obtenus.

27. Kit selon la revendication 26 comprenant ladite première composition et un certain nombre de compositions comprenant chacune un seul variant dudit allergène ns-LTP, égal au nombre des variants dudit allergène ns-LTP.

28. Kit selon la revendication 26 ou 27 où ledit allergène ns-LTP est ParJ1.


### SEQUENCE LISTING

<110> Consiglio Nazionale delle Ricerche
<120> ns-LTPS allergen variants, uses thereof and compositions comprising them
<130> BX1575R
<160> 15
<170> PatentIn version 3.1
<210> 1
   <211> 420
   <212> DNA
   <213> Artificial sequence
<220>
   <223> Mature Parj1.0102 wild type
<220>
   <221> CDS
   <222> (1)..(420)
   <223>
<400> 1
   caa gaa acc tgc ggg act atg gtg aga gcg ctg atg ccg tgc ctg ccg      48
   Gln Glu Thr Cys Gly Thr Met Val Arg Ala Leu Met Pro Cys Leu Pro
   1               5                   10                  15
   ttc gtg cag ggg aaa gag aaa gag ccg tca aag ggg tgc tgc agc ggc      96
   Phe Val Gln Gly Lys Glu Lys Glu Pro Ser Lys Gly Cys Cys Ser Gly
               20                  25                  30
   gcc aaa aga ttg gac ggg gag acg aag acg ggg ccg cag agg gtg cac     144
   Ala Lys Arg Leu Asp Gly Glu Thr Lys Thr Gly Pro Gln Arg Val His
           35                  40                  45
   gct tgt gag tgc atc cag acc gcc atg aag act tat tcc gac atc gac     192
   Ala Cys Glu Cys Ile Gln Thr Ala Met Lys Thr Tyr Ser Asp Ile Asp
   50 55 60
   ggg aaa ctc gtc agc gag gtc ccc aag cac tgc ggc atc gtt gac agc 240
   Gly Lys Leu Val Ser Glu Val Pro Lys His Cys Gly Ile Val Asp Ser
   65 70 75 80
   aag ctc ccg ccc att gac gtc aac atg gac tgc aag aca gtt gga gtg 288
   Lys Leu Pro Pro Ile Asp Val Asn Met Asp Cys Lys Thr Val Gly Val
   85 90 95
   gtt cct cgg caa ccc caa ctt cca gtc tct ctc cgt cat ggt ccc gtc 336
   Val Pro Arg Gln Pro Gln Leu Pro Val Ser Leu Arg His Gly Pro Val
   100 105 110
   acg ggc cca agt gat ccc gcc cac aaa gca cgg ttg gag aga ccc cag 384
   Thr Gly Pro Ser Asp Pro Ala His Lys Ala Arg Leu Glu Arg Pro Gln
   115 120 125
   att aga gtt ccg ccc ccc gca ccg gaa aaa gcc taa 420
   Ile Arg Val Pro Pro Pro Ala Pro Glu Lys Ala
   130 135
<210> 2
   <211> 139
   <212> PRT
   <213> Artificial sequence
<220>
   <223> Mature Parj1.0102 wild type
<400> 2
   Gln Glu Thr Cys Gly Thr Met Val Arg Ala Leu Met Pro Cys Leu Pro
   1 5 10 15
   Phe Val Gln Gly Lys Glu Lys Glu Pro Ser Lys Gly Cys Cys Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Val Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 3
   <211> 420
   <212> DNA
   <213> Artificial Sequence
<220>
   <223> Parj 1 variant PjA
<220>
   <221> CDS
   <222> (1)..(420)
   <223> Coding sequence for the mutant of ParJ1 protein by subst itution o
   f Cys 29 and 30 with Ser
<400> 3
   caa gaa acc tgc ggg act atg gtg aga gcg ctg atg ccg tgc ctg ccg
       48
   Gln Glu Thr Cys Gly Thr Met Val Arg Ala Leu Met Pro Cys Leu Pro
   1               5                   10                  15
   ttc gtg cag ggg aaa gag aaa gag ccg tca aag ggg agc agc agc ggc
       96
   Phe Val Gln Gly Lys Glu Lys Glu Pro Ser Lys Gly Ser Ser Ser Gly
               20                  25                  30
   gcc aaa aga ttg gac ggg gag acg aag acg ggg ccg cag agg gtg cac
       144
   Ala Lys Arg Leu Asp Gly Glu Thr Lys Thr Gly Pro Gln Arg Val His
           35                  40                  45
   gct tgt gag tgc atc cag acc gcc atg aag act tat tcc gac atc gac
       192
   Ala Cys Glu Cys Ile Gln Thr Ala Met Lys Thr Tyr Ser Asp Ile Asp
       50                  55                  60
   ggg aaa ctc gtc agc gag gtc ccc aag cac tgc ggc atc gtt gac agc
       240
   Gly Lys Leu Val Ser Glu Val Pro Lys His Cys Gly Ile Val Asp Ser
   65                  70                  75                  80
   aag ctc ccg ccc att gac gtc aac atg gac tgc aag aca gtt gga gtg
       288
   Lys Leu Pro Pro Ile Asp Val Asn Met Asp Cys Lys Thr Val Gly Val
   85 90 95
   gtt cct cgg caa ccc caa ctt cca gtc tct ctc cgt cat ggt ccc gtc 336
   Val Pro Arg Gln Pro Gln Leu Pro Val Ser Leu Arg His Gly Pro Val
   100 105 110
   acg ggc cca agt gat ccc gcc cac aaa gca cgg ttg gag aga ccc cag 384
   Thr Gly Pro Ser Asp Pro Ala His Lys Ala Arg Leu Glu Arg Pro Gln
   115 120 125
   att aga gtt ccg ccc ccc gca ccg gaa aaa gcc taa 420
   Ile Arg Val Pro Pro Pro Ala Pro Glu Lys Ala
   130 135
<210> 4
   <211> 139
   <212> PRT
   <213> Artificial Sequence
<220>
   <223> Parj 1 variant PjA
<400> 4
   Gln Glu Thr Cys Gly Thr Met Val Arg Ala Leu Met Pro Cys Leu Pro
   1 5 10 15
   Phe Val Gln Gly Lys Glu Lys Glu Pro Ser Lys Gly Ser Ser Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Val Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 5
   <211> 420
   <212> DNA
   <213> Artificial Sequence
<220>
   <223> Parj 1 variant PjB
<220>
   <221> CDS
   <222> (1)..(420)
   <223> Coding sequence for the mutant of ParJ 1 protein by subs titution
   of Cys 50 and 52 with Ser
<400> 5
   caa gaa acc tgc ggg act atg gtg aga gcg ctg atg ccg tgc ctg ccg 48
   Gln Glu Thr Cys Gly Thr Met Val Arg Ala Leu Met Pro Cys Leu Pro
   1 5 10 15
   ttc gtg cag ggg aaa gag aaa gag ccg tca aag ggg tgc tgc agc ggc 96
   Phe Val Gln Gly Lys Glu Lys Glu Pro Ser Lys Gly Cys Cys Ser Gly
   20 25 30
   gcc aaa aga ttg gac ggg gag acg aag acg ggg ccg cag agg gtg cac 144
   Ala Lys Arg Leu Asp Gly Glu Thr Lys Thr Gly Pro Gln Arg Val His
   35 40 45
   gct agt gag agc atc cag acc gcc atg aag act tat tcc gac atc gac 192
   Ala Ser Glu Ser Ile Gln Thr Ala Met Lys Thr Tyr Ser Asp Ile Asp
   50 55 60
   ggg aaa ctc gtc agc gag gtc ccc aag cac tgc ggc atc gtt gac agc 240
   Gly Lys Leu Val Ser Glu Val Pro Lys His Cys Gly Ile Val Asp Ser
   65 70 75 80
   aag ctc ccg ccc att gac gtc aac atg gac tgc aag aca gtt gga gtg 288
   Lys Leu Pro Pro Ile Asp Val Asn Met Asp Cys Lys Thr Val Gly Val
   85 90 95
   gtt cct cgg caa ccc caa ctt cca gtc tct ctc cgt cat ggt ccc gtc 336
   Val Pro Arg Gln Pro Gln Leu Pro Val Ser Leu Arg His Gly Pro Val
   100 105 110
   acg ggc cca agt gat ccc gcc cac aaa gca cgg ttg gag aga ccc cag
   384
   Thr Gly Pro Ser Asp Pro Ala His Lys Ala Arg Leu Glu Arg Pro Gln
   115 120 125
   att aga gtt ccg ccc ccc gca ccg gaa aaa gcc taa
   420
   Ile Arg Val Pro Pro Pro Ala Pro Glu Lys Ala
   130 135
<210> 6
   <211> 139
   <212> PRT
   <213> Artificial Sequence
<220>
   <223> Parj 1 variant PjB
<400> 6
   Gln Glu Thr Cys Gly Thr Met Val Arg Ala Leu Met Pro Cys Leu Pro
   1 5 10 15
   Phe Val Gln Gly Lys Glu Lys Glu Pro Ser Lys Gly Cys Cys Ser Gly
   20 25 30
   Ala Lys Arg Leu Asp Gly Glu Thr Lys Thr Gly Pro Gln Arg Val His
   35 40 45
   Ala Ser Glu Ser Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Val Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 7
   <211> 420
   <212> DNA
   <213> Artificial Sequence
<220>
   <223> Parj1 variant PjC
<220>
   <221> CDS
   <222> (1) .. (420)
   <223> Coding sequence for Parj 1 mutant by substitution of the Cys 4, 2
   9 and 30 with Ser
<400> 7
   caa gaa acc agc ggg act atg gtg aga gcg ctg atg ccg tgc ctg ccg 48
   Gln Glu Thr Ser Gly Thr Met Val Arg Ala Leu Met Pro Cys Leu Pro
   1 5 10 15
   ttc gtg cag ggg aaa gag aaa gag ccg tca aag ggg agc agc agc ggc 96
   Phe Val Gln Gly Lys Glu Lys Glu Pro Ser Lys Gly Ser Ser Ser Gly
   20 25 30
   gcc aaa aga ttg gac ggg gag acg aag acg ggg ccg cag agg gtg cac 144
   Ala Lys Arg Leu Asp Gly Glu Thr Lys Thr Gly Pro Gln Arg Val His
   35 40 45
   gct tgt gag tgc atc cag acc gcc atg aag act tat tcc gac atc gac 192
   Ala Cys Glu Cys Ile Gln Thr Ala Met Lys Thr Tyr Ser Asp Ile Asp
   50 55 60
   ggg aaa ctc gtc agc gag gtc ccc aag cac tgc ggc atc gtt gac agc 240
   Gly Lys Leu Val Ser Glu Val Pro Lys His Cys Gly Ile Val Asp Ser
   65 70 75 80
   aag ctc ccg ccc att gac gtc aac atg gac tgc aag aca gtt gga gtg 288
   Lys Leu Pro Pro Ile Asp Val Asn Met Asp Cys Lys Thr Val Gly Val
   85 90 95
   gtt cct cgg caa ccc caa ctt cca gtc tct ctc cgt cat ggt ccc gtc 336
   Val Pro Arg Gln Pro Gln Leu Pro Val Ser Leu Arg His Gly Pro Val
   100 105 110
   acg ggc cca agt gat ccc gcc cac aaa gca cgg ttg gag aga ccc cag 384
   Thr Gly Pro Ser Asp Pro Ala His Lys Ala Arg Leu Glu Arg Pro Gln
   115 120 125
   att aga gtt ccg ccc ccc gca ccg gaa aaa gcc taa 420
   Ile Arg Val Pro Pro Pro Ala Pro Glu Lys Ala
   130 135
<210> 8
   <211> 139
   <212> PRT
   <213> Artificial Sequence
<220>
   <223> Parj1 variant PjC
<400> 8
   Gln Glu Thr Ser Gly Thr Met Val Arg Ala Leu Met Pro Cys Leu Pro
   1               5                   10                  15
   Phe Val Gln Gly Lys Glu Lys Glu Pro Ser Lys Gly Ser Ser Ser Gly
               20                  25                  30
   Ala Lys Arg Leu Asp Gly Glu Thr Lys Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Lys Leu Val Ser Glu Val Pro Lys His Cys Gly Ile Val Asp Ser
   65                  70                  75                  80
   Lys Leu Pro Pro Ile Asp Val Asn Met Asp Cys Lys Thr Val Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 9
   <211> 420
   <212> DNA
   <213> Artificial Sequence
<220>
   <223> Parj 1 variant PjD
<220>
   <221> CDS
   <222> (1)..(420)
   <223> Coding sequence for Parj 1 mutant by substitution of the
   Cys 29,
   30, 50 and 52 with Ser
<400> 9
   caa gaa acc tgc ggg act atg gtg aga gcg ctg atg ccg tgc ctg ccg 48
   Gln Glu Thr Cys Gly Thr Met Val Arg Ala Leu Met Pro Cys Leu Pro
   1 5 10 15
   ttc gtg cag ggg aaa gag aaa gag ccg tca aag ggg agc agc agc ggc 96
   Phe Val Gln Gly Lys Glu Lys Glu Pro Ser Lys Gly Ser Ser Ser Gly
   20 25 30
   gcc aaa aga ttg gac ggg gag acg aag acg ggg ccg cag agg gtg cac 144
   Ala Lys Arg Leu Asp Gly Glu Thr Lys Thr Gly Pro Gln Arg Val His
   35 40 45
   gct agt gag agc atc cag acc gcc atg aag act tat tcc gac atc gac 192
   Ala Ser Glu Ser Ile Gln Thr Ala Met Lys Thr Tyr Ser Asp Ile Asp
   50 55 60
   ggg aaa ctc gtc agc gag gtc ccc aag cac tgc ggc atc gtt gac agc 240
   Gly Lys Leu Val Ser Glu Val Pro Lys His Cys Gly Ile Val Asp Ser
   65 70 75 80
   aag ctc ccg ccc att gac gtc aac atg gac tgc aag aca gtt gga gtg 288
   Lys Leu Pro Pro Ile Asp Val Asn Met Asp Cys Lys Thr Val Gly Val
   85 90 95
   gtt cct cgg caa ccc caa ctt cca gtc tct ctc cgt cat ggt ccc gtc 336
   Val Pro Arg Gln Pro Gln Leu Pro Val Ser Leu Arg His Gly Pro Val
   100 105 110
   acg ggc cca agt gat ccc gcc cac aaa gca cgg ttg gag aga ccc cag 384
   Thr Gly Pro Ser Asp Pro Ala His Lys Ala Arg Leu Glu Arg Pro Gln
   115 120 125
   att aga gtt ccg ccc ccc gca ccg gaa aaa gcc taa 420
   Ile Arg Val Pro Pro Pro Ala Pro Glu Lys Ala
   130 135
<210> 10
   <211> 139
   <212> PRT
   <213> Artificial Sequence
<220>
   <223> Parj 1 variant PjD
<400> 10
   Gln Glu Thr Cys Gly Thr Met Val Arg Ala Leu Met Pro Cys Leu Pro
   1               5                   10                  15
   Phe Val Gln Gly Lys Glu Lys Glu Pro Ser Lys Gly Ser Ser Ser Gly
               20                  25                  30
   Ala Lys Arg Leu Asp Gly Glu Thr Lys Thr Gly Pro Gln Arg Val His
           35                  40                  45
   Ala Ser Glu Ser Ile Gln Thr Ala Met Lys Thr Tyr Ser Asp Ile Asp
       50                  55                  60
   Gly Lys Leu Val Ser Glu Val Pro Lys His Cys Gly Ile Val Asp Ser
   65                  70                  75                  80
   Lys Leu Pro Pro Ile Asp Val Asn Met Asp Cys Lys Thr Val Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala
       130                 135
<210> 11
   <211> 30